(19) Europäisches Patentamt
European Patent Office
Office européen des brevets

(11) EP 2 848 607 A1

(12) EUROPÄISCHE PATENTANMELDUNG

(43) Veröffentlichungstag:
**18.03.2015 Patentblatt 2015/12**

(51) Int Cl.:
*C07C 319/20* (2006.01) *C07C 319/26* (2006.01)
*C07C 319/28* (2006.01) *C07C 323/58* (2006.01)

(21) Anmeldenummer: **13184831.9**

(22) Anmeldetag: **17.09.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Evonik Industries AG**
**45128 Essen (DE)**

(72) Erfinder:
- **Reichert, Stefan, Dr.**
  **60439 Frankfurt (DE)**
- **Jakob, Harald, Dr.**
  **63594 Hasselroth (DE)**
- **Hasselbach, Hans Joachim, Dr.**
  **63571 Gelnhausen (DE)**
- **Körfer, Martin**
  **63796 Kahl (DE)**

(54) **Verfahren zur Gewinnung von Methionin**

(57) Die Erfindung bezieht sich auf ein Verfahren zur Kristallisation von D,L-Methionin aus D,L-Methionin und D,L-Methioninammoniumsalze enthaltenden wässrigen Lösungen und/oder Suspensionen mit einem Met-Gehalt von 70 - 180 g/kg, einem $NH_4^+$-Gehalt von 1 - 5 g/kg in Gegenwart eines Kristallisationsadditivs, welches ein nichtionisches oder anionisches Tensid oder eine Mischung aus verschiedenen nichtionischen oder anionischen Tensiden umfasst, bei dem die Temperatur der Lösung und/oder Suspension von $T_1$ = 85 - 110°C direkt oder schrittweise auf $T_2$ = 30 - 50°C abgesenkt wird, so dass D,L-Methionin als Feststoff ausfällt.

EP 2 848 607 A1

## Beschreibung

## Stand der Technik

Verfahren zur Herstellung von D, L-Methionin, bei denen das D,L-Methionin zunächst als Ammoniummethioninat anfällt, sind bekannt.

Nach US20050176115 wird eine wässrige, ammoniumhaltige D,L-Methioninlösung erhalten, indem wässrige Lösungen von 2-Amino-4-methylthiobutyronitril bereits in Gegenwart von $NH_3$ mit Biokatalysatoren zum D,L-Methionin umgesetzt werden. Das D,L-Methionin wird anschließend durch Abtrennen des Ammoniaks bei verringertem Druck ausgefällt. Das so erhaltene D,L-Methionin weist eine Reinheit von 99% auf. Das Schüttgewicht des erhaltenen D,L-Methionins wird nicht angegeben.

In JP2004-254690 wird ebenfalls durch Umsetzung von 2-Amino-4-methylthiobutyronitril mit einem Biokatalysator D,L-Methionin und $NH_3$ gebildet. Durch die Gegenwart des Ammoniaks wird die Löslichkeit des Methionins erhöht, was die Abtrennung des Biokatalysators erleichtert. Über die genauen Isolierungsbedingungen sowie die Produkteigenschaften des D,L-Methionins wird in JP2004-254690 keine Aussage gemacht.

DE 60127538 beschreibt ein Verfahren bei dem D,L-Methionin durch eine katalysierte Verseifungsreaktion aus Methioninamid erzeugt wird. Aus der entstandenen Ammoniummethioninatlösung wird hier der Ammoniak durch Strippen vollständig entfernt und das Methionin auskristallisiert, wobei keine Schüttgewichte des erhaltenen Methionins angegeben werden.

In WO 2008006977 wird D,L-Methionin über eine Verseifung mit $NH_3$ aus dem Methioninhydantoin gewonnen und durch Abdampfen von $NH_3$ und $CO_2$ bei vermindertem Druck wird das D,L-Methionin gewonnen. Über die Produkteigenschaften wird nichts gesagt.

WO2007034065 beschreibt ebenfalls eine ammoniakalische Verseifung von Methioninhydantoin. Aus der Ammoniummethioninatlösung wird der Ammoniak in einer Strippkolonne entfernt und anschließend das Methionin durch abkühlen der Lösung ausgefällt. Es findet sich keine Aussage zu den Produkteigenschaften des gebildeten Methionins.

DE 10238212 beschreibt ein Verfahren bei dem Methioninhydantoin in Wasser mit oder ohne Katalysator bei hohen Temperaturen verseift wird. Vor der Kristallisation des entstandenen Methionins wird $NH_3$ und $CO_2$ teilweise abgetrennt. Es werden weder die Restmengen an $CO_2$ und Ammoniak in der Lösung aus der die Kristallisation erfolgt genannt, noch wird etwas über das Schüttgewicht des erhaltenen Methionins ausgesagt.

Die WO 2003050071 beschreibt wässrige Mischungen von Fettsäurepolyethylenglycolester mit modifizierten Cellulosen, die als Hilfsmittel bei der Kristallisation von Methionin aus mit Kohlendioxid neutralisierten Kaliummethioninatlösungen aus der alkalischen Methionin-Hydantoin-Verseifung verwendet werden. Bei diesem speziellen Verfahren in Gegenwart großer Kaliummengen werden Schüttgewichte von bis zu 586g/l, in einem Fall von 620g/l, erhalten (mit Additiv Hydroxyethylcellulose). Es werden jedoch keine Angaben gemacht zur Kristallisation von D,L-Methionin aus D,L-Ammoniummethioninat enthaltenden Lösungen.

Die Löslichkeit von D,L-Methionin wird in Gegenwart von $NH_3$ erhöht (siehe Löslichkeitskurven aus JP2004-254690).

Für ein wirtschaftliches Verfahren zur Isolierung von D, L-Methionin ist es demnach zweckmässig, den Ammoniak möglichst vollständig zu entfernen, um die Menge an kristallisierbarem D,L-Methionin zu maximieren. Hierzu kann der Ammoniak durch verschiedene literaturbekannte Verfahren wie Strippen, Abdampfen bei vermindertem Druck etc. entfernt werden.

## Aufgabe der Erfindung

Die Aufgabe der Erfindung ist es eine Methode zur Isolierung von D, L-Methionin ausgehend von Ammoniummethioninat enthaltenden Lösungen bereitzustellen, bei der das D,L-Methionin mit hohen Schüttgewichten > 550 g/l erhalten wird.

## Beschreibung

Bei den vorliegenden Untersuchungen hat sich gezeigt, dass das D,L-Methionin bei einer Kristallisation aus wässrigen Lösungen üblicherweise mit niedrigen Schüttgewichten < 550 g/l erhalten wird. Durch die alleinige Zugabe von Kristallisationsadditiven konnte keine signifikante Erhöhung des Schüttgewichtes erreicht werden. Überraschenderweise hat sich gezeigt, dass eine Restmenge $NH_3$ in Kombination mit zugesetzten Additiven zu D,L-Methionin mit höherem Schüttgewicht führt.

Da sich in wässrigen Lösungen, die sowohl $NH_3$ als auch Methionin enthalten ein Gleichgewicht zwischen Ammoniummethioninat einerseits und Methionin und Ammoniak andererseits einstellt, wird zur Vereinfachung im Folgenden nur noch über $NH_4^+$-Konzentrationen gesprochen, unabhängig davon, ob es sich dabei um $NH_4^+$ oder $NH_3$

handelt.

Die oben genannte Aufgabe sowie weitere damit im Zusammenhang stehende aber nicht explizit genannte Aufgaben werden gelöst, durch Bereitstellung eines Verfahrens zur Kristallisation von D,L-Methionin aus D,L-Methionin und D,L-Methioninammoniumsalz enthaltenden wässrigen Lösungen und/oder Suspensionen mit einem Met-Gehalt (umfassend das gesamte Methionin in Form von D,L-Methionin und D,L-Methioninammoniumsalz) von 70 - 180 g/kg der Lösung und/oder Suspension (7 - 18 Gew.-%), vorzugsweise 90 - 150 g/kg (9 - 15 Gew.-%), einem $NH_4^+$-Gehalt von 1 - 5 g/kg der Lösung und/oder Suspension (0,1 - 0,5 Gew.-%), vorzugsweise 1,5 bis 3,0 g/kg (0,15 - 0,3 Gew.%), in Gegenwart eines Kristallisationsadditivs,

welches ein nichtionisches oder anionisches Tensid oder eine Mischung aus verschiedenen nichtionischen oder anionischen Tensiden umfasst,

bei dem die Temperatur der Lösung und/oder Suspension von einem Temperaturbereich $T_1$ = 85 - 110°C direkt oder schrittweise auf einen Temperaturbereich $T_2$ = 30 - 50°C abgesenkt wird, so dass Methionin als Feststoff aus der Lösung und/oder Suspension ausfällt.

Unter D,L-Methionin und D,L-Methioninammoniumsalz enthaltender wässriger Lösung wird hier verstanden, dass der überwiegende Anteil des gesamten Methionins (Met) gelöst vorliegt und nur geringe Met-anteile von max. 5% ungelöst, also suspendiert vorliegen.

Unter D,L-Methionin und D,L-Methioninammoniumsalz enthaltender wässriger Suspension wird hier verstanden, dass ein deutlicher Anteil nämlich von > 5% des gesamten Methionins suspendiert vorliegt, während der Rest in gelöster Form vorliegt.

Entsprechend liegt die optimale $NH_4^+$-Konzentration bezogen auf Methionin bei mindestens ca. 5 g $NH_4^+$ / kg Met und bei maximal ca. 60 g $NH_4^+$ / kg Met wenn die Methioninkonzentration in der Lösung im Bereich von 90 g/kg bis 150 g/kg liegt.

Die $NH_4^+$-Konzentration kann dabei beispielsweise mit einer $NH_4^+$-sensitiven Elektrode nach bekannten Verfahren bestimmt werden. Die $NH_4^+$-Konzentration wird hier in der Regel durch Messung an einer auf pH 11 eingestellten Probenlösung und Vergleich mit der Messung an ebenfalls auf pH 11 eingestellten $NH_4Cl$-Lösungen bekannter Konzentration ermittelt.

Die Methioninkonzentration in der Lösung und/oder Suspension wird am einfachsten per HPLC bestimmt.

Durch die beschriebene Kombination aus der Gegenwart von $NH_4^+$-Ionen, der Zugabe von Kristallisationsadditiv und der Temperatursteuerung der Kristallisation werden mit dem erfindungsgemäßen Verfahren grobkörnige, gut filtrierbare Methioninkristalle mit einem Schüttgewicht von > 550 g/l nach Trocknung erhalten.

Als anionische Tenside eignen sich insbesondere Tenside nach einer der in Formel 1 bis 3 dargestellten Verbindungen, oder Mischungen daraus:

$R^1$-O-$SO_3$M (Formel 1)

$R^2$-O-$(CH_2)_n$-$SO_3$M (Formel 2)

$R^3$-$(O\text{-}C_2H_4)_n$-O-$SO_3$M (Formel 3)

wobei n eine ganze Zahl von 1 bis 10, M Natrium oder Kalium und $R^1$, $R^2$ und $R^3$ eine lineare, verzweigte oder cyclische, gesättigte oder ungesättigte $C_8$- bis $C_{20}$-Alkylgruppe oder eine Arylgruppe ist. Mit diesen wurden, wie die Beispiel 1 mit den Zusätzen 2, 3 und 4 in Tabelle 1 zeigt, relativ hohe Schüttgewichte von 564 bis 588 kg/l erhalten.

Vorzugsweise verwendet werden Tenside, bei denen n gleich 2 und $R^1$, $R^2$ und $R^3$ lineare, gesättigte $C_8$- bis $C_{18}$- Alkylgruppen sind, weil diese kommerziell leicht erhältlich und wirksam sind.

Besonders bevorzugt eingesetzt werden anionische Tenside, der Formeln $C_nH_{2n+1}$-O-$SO_3$Na, mit n = 12 bis 18 (Sulfopon® 1218G, Oleochemicals) $C_nH_{2n+1}$-O-$C_2H_4$-$SO_3$Na, mit n = 8 bis 18 (Hostapon® SCI 85, Clariant) $C_nH_{2n+1}$-$(OC_2H_4)_2$-O-$SO_3$Na, mit n = 12 (Disponil® FES 27, Cognis)

In einer bevorzugten Ausführungsform des Verfahrens wird als nichtionisches Tensid ein Sorbitanfettsäureester oder eine Mischung aus verschiedenen Sorbitanfettsäureestern, besonders bevorzugt polyethoxylierte Sorbitanfettsäureester verwendet. Sorbitanfettsäureester haben den Vorteil, dass sie wirksam und kommerziell leicht erhältlich sind. In einer ganz besonders bevorzugten Ausführungsform ist das nichtionische Tensid ein polyethoxyliertes Sorbitantristearat gemäß der Formel 4:

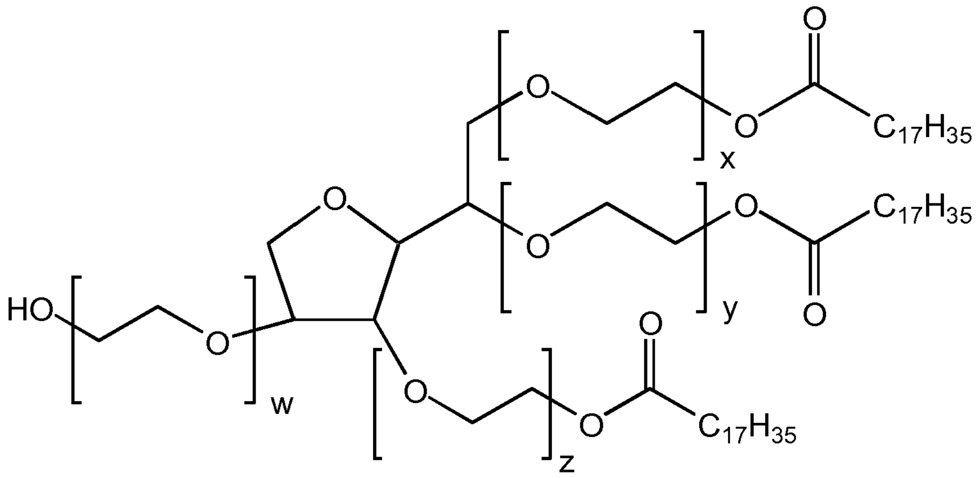

(Formel 4)

wobei w + x + y+ z = 20.

Mit diesen wurden, wie das Beispiel 3 zeigt, ein relativ hohes Schüttgewicht von 578 kg/l erhalten.

Die Konzentration des Kristallisationsadditivs (auf Basis des Wirkstoffs) in der Lösung und/oder Suspension, aus der die Kristallisation erfolgt, beträgt vorzugsweise mindestens 700 ppm und maximal 4000 ppm bezogen auf die Gesamtmasse der Lösung und/oder Suspension, besonders bevorzugt mindestens 750 ppm und maximal 2000 ppm, am meisten besonders bevorzugt mindestens 800 ppm und maximal 1000 ppm. Dies gewährleistet eine gute Entfaltung der Wirkung des Additivs ohne zu viel an Fremdstoffen in die Produktlösung einzutragen.

Um eine optimale Dosierung und Verteilung des Kristallisationsadditivs zu erreichen, wird dieses vorzugsweise in Form einer wässrigen Lösung oder Emulsion eingesetzt, wobei die Konzentration des Kristallisationsadditivs in der Lösung oder Emulsion bevorzugt 2 bis 15 Gewichts-% beträgt.

In einer bevorzugten Ausführungsform des erfindungsgemäßen Verfahrens enthält die Lösung, aus der die Kristallisation erfolgt, zusätzlich einen Entschäumer. Der Entschäumer hat die Funktion den bei der Handhabung der Methioninlösung und/oder -suspension entstehenden und von einigen der oben genannten Kristallisationsadditive verstärkten bzw. verursachten Schaum niederzuschlagen. Darüber hinaus ergibt sich bei einem gleichzeitigen Einsatz von Entschäumer und Kristallisationsadditiven überraschenderweise ein synergistischer Effekt bei den erzielten Schüttgewichten des Methionins, wodurch Schüttgewichte von sogar über 600 g/l erreicht und gleichzeitig die negativen Auswirkungen von Anreicherungsprozessen vermieden werden und damit das erfindungsgemäße Verfahren auch in kontinuierlicher Verfahrensweise durchführbar ist. Dies zeigt sich insbesondere durch Vergleich der Versuche 2, 3 bzw. 4 (ohne zusätzlichen Entschäumer -> Schüttgewichte von 564-588 kg/l)) mit den Versuchen 8, 9 bzw. 10 (mit zusätzlichem Entschäumer -> Schüttgewichte von 630- 634 kg/l) in Tabelle 1 von Beispiel 1. Zusätzlich ermöglicht diese Ausführungsform weniger Kristallisationsadditiv einzusetzen.

Vorzugsweise eingesetzt werden Entschäumer, die Silikonöl enthalten, da diese sich als besonders effektiv erwiesen haben, wobei bevorzugt ein Silikonöl mit einer kinematischen Viskosität von 0,65 bis 10000 $mm^2/s$ (gemessen bei 25°C gemäß DIN 53018), besonders bevorzugt von 90 bis 1500 $mm^2/s$ eingesetzt wird. Der Entschäumer kann weiterhin Bestandteile enthalten, die als Emulgatoren wirksam sind, beispielsweise Mischungen polyethoxylierter Fettsäuren und polyethoxylierter Fettalkohole. Der Entschäumer kann ebenfalls Kieselsäure enthalten. In einer bevorzugten Ausführungsform ist der Entschäumer eine wässrige Lösung, die 5 bis 10 Gewichts-% Silikonöl, 0,05 bis 1 Gewichts-% Kieselsäure, 0,5 bis 5 Gewichts-% einer Mischung polyethoxylierter Fettsäuren, sowie 2 bis 7 Gewichts-% einer Mischung polyethoxylierter Fettalkohole enthält.

Vorzugsweise wird der Entschäumer in einer Mischung mit dem Kristallisationsadditiv eingesetzt. Um eine kontinuierliche, stabile Dosierung des Entschäumers zu erreichen, wird er vorzugsweise vor seinem Einsatz noch weiter mit Wasser verdünnt.

Die Verwendung von Silikonölentschäumern führt dazu, dass in dem nach dem erfindungsgemäßen Verfahren hergestellten Methionin mit einem geeigneten Analysenverfahren (z. B. Röntgenphotoelektronenspektroskopie, kurz XPS) Silicium nachgewiesen werden kann.

Bevorzugt verwendet wird der Entschäumer im erfindungsgemäßen Verfahren dabei so, dass das Gewichtsverhältnis des Entschäumers: Kristallisationsadditiv (auf Basis des Wirkstoffs) in der Lösung bzw. Suspension, aus der die Kristallisation erfolgt, im Bereich von 4:1 bis 1:1, vorzugsweise im Bereich von 3:1 bis 2:1 liegt und dabei die Konzentration des Kristallisationsadditivs (auf Basis des Wirkstoffs) mindestens 50 ppm und maximal 1200 ppm bezogen auf die Gesamtmasse der Lösung und/oder Suspension beträgt, vorzugsweise 100 ppm bis 600 ppm, besonders bevorzugt 200 ppm bis 400 ppm. Die dabei erzielten hohen D,L-Methionin-Schüttgewichte von deutlich über 600 g/l lassen

sich besonders gut in Beispiel 4/Tabelle 3 erkennen.

Das erfindungsgemäße Verfahren wird dabei vorzugsweise so geführt, dass die Kristallisation durch Einleiten einer 85 bis 110°C heißen D,L-Methionin und D,L-Methioninammoniumsalz enthaltenden wässrigen Lösung und/oder Suspension in eine 30 bis 50°C warme D,L-Methionin und D,L-Methioninammoniumsalz enthaltenden wässrigen Lösung und/oder Suspension erfolgt, wobei die Temperatur der dabei entstehenden Mischung konstant zwischen 30 und 50°C gehalten wird.

Ganz besonders bevorzugt ist es dabei, eine 85 bis 110°C heiße D,L-Methionin- und D,L-Methioninammoniumsalz enthaltende wässrige Lösung in eine 30 bis 50°C warme D,L-Methionin und D,L-Methioninammoniumsalz enthaltende wässrige Suspension einzuleiten. Dies hat den Vorteil, dass besonders gut filtrierbare Kristalle erhalten werden.

Eine weitere bevorzugte Ausführungsform des Verfahrens ist dadurch gekennzeichnet, dass die Kristallisation zweistufig durchgeführt wird, wobei in der ersten Kristallisationsstufe eine 85 bis 110°C heiße D,L-Methionin und D,L-Methioninammoniumsalz enthaltende Lösung und/oder Suspension in eine 60 bis 80 °C warme D,L-Methionin und D,L-Methioninammoniumsalz enthaltende Suspension eingeleitet und die Temperatur der dabei entstehenden Mischung konstant zwischen 60 und 80 °C gehalten wird und wobei die in der ersten Kristallisationsstufe erhaltene 60 bis 80 °C warme D,L-Methionin und D,L-Methioninammoniumsalz enthaltende Suspension in einer zweiten Kristallisationsstufe in eine 30 bis 50 °C warme D,L-Methionin und DL-Methioninammoniumsalz enthaltende Suspension eingeleitet wird, wobei die Temperatur der dabei entstehenden Mischung konstant zwischen 30 und 50 °C gehalten wird. Dabei kann der Anteil an Verunreinigungen in der Kristallisationsbrühe in besonders effektiver Weise kontrolliert bzw. durch Ausschleusung an geeigneter Stelle abgereichert werden, ohne dass der vorteilhafte Effekt des Schüttgewichtes über 550 g/l aufgegeben wird.

Eine außerdem bevorzugte Ausführungsform des erfindungsgemäßen Verfahrens ist dadurch gekennzeichnet, dass die Kristallisation durch Vakuumkristallisation erfolgt, wobei der Druck in der ersten Kristallisationsstufe 60 bis 1000 mbar und, falls eine zweistufige Kristallisation durchgeführt wird, in der zweiten Kristallisationsstufe 35 bis 200 mbar beträgt. Dies hat den Vorteil, dass weniger kalte Oberflächen eingesetzt werden. Kalte Oberflächen können zu unerwünschten lokalen Anbackungen führen.

Die für das erfindungsgemäße Kristallisationsverfahren verwendeten D,L-Methionin- und D,L-Methioninammoniumsalze enthaltenden wässrigen Lösungen und/oder Suspensionen können zuvor durch Auflösen und/oder Suspendieren von D,L-Methionin in Wasser in Gegenwart von entsprechenden Mengen Ammoniak hergestellt werden. Das Methionin kann aus einem beliebigen Herstellungsprozess stammen, was das Verfahren universell einsetzbar macht.

Als vorteilhaft dabei hat sich erwiesen, dass zum Auflösen D,L-Methionin mit einem Methioningehalt von mindestens 90 Gew.-%, vorzugsweise mindestens 95 Gew.-%, verwendet wird.

Geeignet dafür ist insbesondere D,L-Methionin in Form von Reinmethionin und/oder Rohmethionin aus einem beliebigen Herstellungsprozess mit einer Restfeuchte von 0,1 bis 9,5 Gew.-%, vorzugsweise 0,2 bis 4,5 Gew.-% . Insbesondere die Verwendung von noch filterfeuchten Rein- bzw. Roh-Methioninqualitäten aus einem technischen Prozess zur Herstellung von D,L-Methionin ist vorteilhaft, weil so direkt D,L-Methionin mit den gewünschten Eigenschaften insbesondere dem Schüttgewicht > 550 kg/l am Ende des Prozesses nach Trocknung erhalten werden kann.

Das erfindungsgemäße Verfahren eignet sich auch besonders für die Isolierung von D,L-Methionin aus D,L-Methionin und D,L-Methioninammoniumsalze enthaltenden wässrigen Lösungen und/oder Suspensionen, welche durch Hydrolyse von D,L-Methioninnitril und/oder D,L-Methioninamid hergestellt worden sind und zwar ohne Verwendung von salzbildenden sauren oder basischen Verseifungsagentien (wie zum Beispiel HCl, $H_2SO_4$ oder Alkalihydroxide wie NaOH oder KOH) mit Ausnahme von Ammoniak. Solche nichtsalzbildende Verseifungsagentien bzw. Verseifungskatalysatoren wie z.B. $TiO_2$ oder $MnO_2$ sind aus der einschlägigen Patentliteratur bekannt und führen ebenso wie Ammoniak als Verseifungsagens in der Verseifung von D,L-Methioninnitril und/oder D,L-Methioninamid direkt zu D,L-Methionin und D,L-Methioninammoniumsalze enthaltenden wässrigen Lösungen und/oder Suspensionen, aus denen D,L-Methionin nach dem erfindungsgemäßen Verfahren durch Kristallisation gewonnen werden kann. Solche wässrigen Lösungen und/oder Suspension enthalten daher praktisch keine Alkali-Ionen, wie zum Beispiel $Na^+$- bzw. $K^+$-Ionen.

Demgemäß ist ein weiterer Gegenstand der Erfindung ein Verfahren zur Herstellung von D,L-Methionin, bei dem zunächst durch Hydrolyse von D,L-Methioninnitril und/oder D,L-Methioninamid eine wässrige Lösung von D,L-Methioninammoniumsalz gebildet wird und daraus durch teilweise Entfernung des Ammoniaks aus dem D,L-Methioninammoniumsalz, welcher darin gebunden als Ammonium-Ion vorliegt, eine D,L-Methionin und D,L-Methioninammoniumsalz enthaltende wässrige Lösung und/oder Suspension erzeugt wird, aus der dann D,L-Methionin durch das erfindungsgemäße Kristallisationsverfahren gewonnen wird.

Bei dem erfindungsgemäßen Verfahren wird die primär gebildete Ammoniummethioninatlösung also zunächst einer Ammoniakabreicherung unterzogen. Verfahren hierzu sind bekannt: Dies kann zum Beispiel über Erhitzen bei vermindertem Druck oder über Strippen mit Dampf erreicht werden.

Demgemäß wird das erfindungsgemäße Verfahren zur Herstellung von D,L-Methionin bevorzugt so durchgeführt, dass die wässrige Lösung von D,L-Methioninammoniumsalz vor der Kristallisation durch Eindampfen und/oder durch Abstrippen von Ammoniak auf einen $NH_4^+$-Gehalt von 1 - 5 g/kg der Lösung und/oder Suspension, vorzugsweise

von 1,5 bis 3,0 g/kg, gebracht wird, z.B. über Erhitzen bei vermindertem Druck oder über Strippen mit Dampf.

Dabei wird erfindungsgemäß die Ammoniumkonzentration aber nur auf Werte in einem Bereich von ca. 5 bis ca. 60 g $NH_3$/kg Methionin abgereichert. Dabei ist es vorteilhaft die Temperatur so hoch zu wählen, dass während des Entfernens von Ammoniak nicht bereits D,L-Methionin ausfällt, sondern in Lösung verbleibt. Die heiße Methioninlösung wird vorzugsweise durch Einleiten in eine vorgelegte, kühlere Methioninsuspension rasch abgekühlt, wodurch eine Überkonzentration von gelöstem D,L-Methionin erzeugt wird und D,L-Methionin aus der Lösung ausfällt.

Dabei kann auch eine Verfahrensweise angewendet werden, bei der die wässrige Lösung und/oder Suspension von D,L-Methionin und/oder D,L-Methioninammoniumsalz durch Zugabe von Wasser und/oder D,L-Methionin auf den geeigneten Met-Gehalt von 70 - 180 g/kg der Lösung, vorzugsweise von 90 bis 150 g/kg der Lösung, gebracht wird, was das Verfahren variabel und flexibel verwendbar macht.

Das ausgefallene D,L-Methionin wird bevorzugt von der dabei entstandenen Mutterlauge getrennt und getrocknet oder zunächst umkristallisiert und nach Abtrennung von der dabei entstehenden Mutterlauge getrocknet, wobei schließlich D,L-Methionin mit einer Reinheit von mindestens 99 Gew. % und einem Schüttgewicht von mindestens 550 g/l nach Trocknung erhalten wird.

Die dabei erhaltene Mutterlauge wird vorzugsweise in eine Kristallisationsstufe zurückgeführt, was zur Minimierung von Verlusten an Methionin führt.

Das Verfahren kann sowohl kontinuierlich als auch diskontinuierlich (im batch) durchgeführt werden.

Figur 1 zeigt beispielhaft und schematisch die kontinuierliche Durchführung des erfindungsgemäßen Verfahrens. Zunächst wird die Ammoniummethioninatlösung in eine geeignete Apparatur A zur Reduktion der Ammoniakkonzentration geleitet. Dabei handelt es sich in der Regel um ein Eindampfsystem, z. B. enthaltend einen Fallfilm- oder Umlaufverdampfer. Hier werden die Bedingungen so gewählt, dass im Produktstrom eine $NH_3$ Menge von 1 bis 5 g/kg der Lösung und/oder Suspension enthalten ist, während die Met-Konzentration in einem Bereich von 70 - 180 g/kg der Lösung und/oder Suspension, bevorzugt 90 und 150 g/kg liegt. Diesem Produktstrom wird kontinuierlich das erfindungsgemäße Kristallisationsadditiv ggf. inklusive Entschäumer zugesetzt. Die Temperatur der D,L-Methionin und/oder D,L-Methioninammoniumsalz enthaltenden Lösung und/oder Suspension beträgt bevorzugt 90 bis 100 °C. Diese Met-Lösung und/oder Suspension kann bei Bedarf über einen oder mehrere Wärmetauscher B auf 100 bis 110°C erwärmt und anschließend in einer geeigneten Kristallisationsapparatur C vorzugsweise schnell, ein- oder mehrstufig auf Temperaturen zwischen 30 und 50°C abgekühlt werden, wobei das D,L-Methionin auskristallisiert. Bei Bedarf kann die D,L-Methioninsuspension in einen Zwischenbehälter D geleitet werden, um ein Nachfällen von D,L-Methionin zu ermöglichen. Das D,L-Methionin wird schließlich in einem geeigneten Fest/Flüssigtrennschritt E, wie z. B. einer Filtration oder Zentrifugation, isoliert, wobei das dabei gewonnene Filtrat bei Bedarf in den Zulauf zu Apparatur A zurückgeführt werden kann. Dabei kann es zu einer Anreicherung der erfindungsgemäßen Additive kommen.

Die nachfolgenden Beispiele sollen die Erfindung näher erläutern ohne diese jedoch zu beschränken.

**Beispiele:**

Beispiel 1: Additivscreening anionische Tenside

In einem Kolben wurden 40 g D,L-Methionin und 360 g Wasser vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 125 g D,L-Methionin in 1125 g Wasser gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge in ml bestimmt, das D,L-Methionin abfiltriert und mit 300 ml Aceton gewaschen. Nach Trocknung des D,L-Methionins wurde das Schüttgewicht bestimmt.

Für die Versuche mit $NH_3$ wurde die gewünschte Konzentration als $NH_4^+$-Konzentration berechnet und in beiden Startlösungen/-suspensionen eingestellt. Zusätzlich wurde die Methioninmenge äquimolar zur zugegebenen $NH_3$-Menge erhöht.

Die Kristallisationsversuche wurden in Gegenwart der folgenden Zusätze durchgeführt, wobei die angegebene Konzentration durch Zugabe des Additivs in beiden Startlösungen/-suspensionen eingestellt wurde.

Zusatz 1

Als reiner Entschäumer 1 (Zusatz 1, Vergleichsbeispiel) wurde eine wässrige Mischung enthaltend 6,9 Gewichts-% Silikonöl mit einer kinematischen Viskosität von 1000 $mm^2/s$ (AK 1000, Wacker-Chemie GmbH), 0,27 Gewichts-% hydrophobierter Kieselsäure (Sipernat D10, Evonik Degussa GmbH) und 17,9 Gewichts-% eines polyethoxylierten Fettsäuregemisches (Intrasol® FS 18/90/7, Ashland Deutschland GmbH) eingesetzt.

Als reine Kristallisationsadditive wurden die folgenden anionischen Tenside eingesetzt:

Zusatz 2) $C_nH_{2n+1}$-O-$SO_3$Na, mit n = 12 bis 18 (Sulfopon® 1218G, Oleochemicals) Zusatz 3) $C_nH_{2n+1}$-O-$C_2H_4$-$SO_3$Na, mit n = 8 bis 18 (Hostapon® SCI 85, Clariant) Zusatz 4) $C_nH_{2n+1}$-$(OC_2H_4)_2$-O-$SO_3$Na, mit n = 12 (Disponil® FES 27, Cognis) Vergleichsbeispiel, Zusatz 5) $C_nH_{2n+1}$-$(OC_2H_4)_{12}$-O-$SO_3$Na, mit n = 12 (Disponil® FES 993, Cognis)

Vergleichsbeispiel, Zusatz 6) $C_nH_{2n+1}$-$(OC_2H_4)_{30}$-O-$SO_3$Na, mit n = 12 (Disponil® FES 77, Cognis)

Für die Kombination der Kristallisationsadditive mit einem Entschäumer wurde eine wässrige Mischung 7 enthaltend 6,1 Gewichts-% Silikonöl mit einer kinematischen Viskosität von 1000 $mm^2/s$ (AK 1000, Wacker-Chemie GmbH), 0,25 Gewichts-% hydrophobierter Kieselsäure (Sipernat D10, Evonik Degussa GmbH), 2,6 Gewichts-% eines polyethoxylierten Fettsäuregemisches (Intrasol® FS 18/90/7, Ashland Deutschland GmbH), 3,7 Gewichts-% eines polyethoxylierten Fettalkoholgemisches (2,35 Gewichts-% Marlipal®, Sasol Germany GmbH, 1,35 Gewichts-% Brij C2, Croda Chemicals Europe) in Wasser eingesetzt (entspricht 12,65 Gewichts-% Wirkstoff). Diese Mischung wurde mit jeweils 5,1 Gewichts-% des entsprechenden Kristallisationsadditivs (2, 3 bzw. 4) in Wasser (entspricht 17,75 Gewichts-% Gesamtwirkstoff, zusammen mit Wasser 100 Gewichts-%) eingesetzt. Folgende Zusätze wurden eingesetzt:

$$\text{Zusatz 8)} = (7) + (2)$$

$$\text{Zusatz 9)} = (7) + (3)$$

$$\text{Zusatz 10)} = (7) + (4)$$

Das Verhältnis von Entschäumer 7: Kristallisationsadditiv (2, 3, 4) betrug jeweils 2,5:1 (auf Basis des Wirkstoffs). Die Konzentrationsangabe in Tabelle 1 gibt den Gesamtwirkstoffgehalt des Zusatzes ohne Wasser bezogen auf die Gesamtmasse der Lösung bzw. Suspension an.

Tabelle 1:

| Zusatz | Zusatz-Konzentration (ppm) 1) | $NH_4^+$-Konzentration (g/kg) 1) | Schaummenge (ml) | D,L-Methionin-Schüttgewicht (g/l) |
|---|---|---|---|---|
| Kein | - | - | 550 | 453 |
| 1 (Vergleichsbeispiel) | 800 | 1 | 20-30 | 454 |
| 2 | 800 | 2,5 | 85 | 564 |
| 3 | 800 | 2,5 | 400 | 573 |
| 4 | 800 | 2,5 | 250 | 588 |
| 5 (Vergleichsbeispiel) | 800 | 2,5 | 800 | 492 |
| 6 (Vergleichsbeispiel) | 800 | 2,5 | 1000 | 463 |
| 8 | 800 | 2,5 | 0 | 630 |
| 9 | 800 | 2,5 | 0 | 634 |
| 10 | 800 | 2,5 | 20-30 | 630 |
| 1)in der Lösung und/oder Suspension (analog Beispiel 1) | | | | |

Beispiel 2: Einfluss der $NH_4^+$-Konzentration auf das Schüttgewicht von Methionin

In einem Kolben wurden 40 g Methionin und 360 g Wasser vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 125 g Methionin in 1125 g Wasser gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das Methionin abfiltriert und mit 300 ml

Aceton gewaschen. Nach Trocknung des Methionins wurde das Schüttgewicht bestimmt.

Für die Versuche mit $NH_3$ wurde die gewünschte Konzentration als $NH_4^+$-Konzentration berechnet und in beiden Startlösungen/-suspensionen durch Zugabe von wässriger $NH_3$-Lösung eingestellt.

Die Kristallisationsversuche wurden in Gegenwart des Zusatzes 8 (gemäß Beispiel 1) durchgeführt, wobei die angegebene Konzentration durch Zugabe des Zusatzes ebenfalls in beiden Startlösungen/-suspensionen gemäß Tabelle 2 eingestellt wurde.

Die Konzentrationsangabe in Tabelle 2 gibt den Gesamtwirkstoffgehalt des Zusatzes ohne Wasser bezogen auf die Gesamtmasse der Lösung bzw. Suspension an.

Tabelle 2:

| Nr. | Zusatz | Zusatz-Konzentration (ppm) 1) | $NH_4^+$-Konzentration (g/kg) 1) | Schaummenge (ml) | D,L-Methionin-Schüttgewicht (g/l) |
|---|---|---|---|---|---|
| 1 | Kein (Vergleich) | - | - | 350 | 434 |
| 2 | 8 (Vergleich) | 800 | - | 200 | 220 |
| 3 | 8 (Vergleich) | 800 | 0,25 | 0 | 420 |
| 4 | 8 (Vergleich) | 800 | 0,5 | 0 | 505 |
| 5 | 8 | 800 | 1 | 0 | 586 |
| 6 | 8 | 800 | 2,5 | 0 | 615 |
| 1)in der Lösung und/oder Suspension (analog Beispiel 1) | | | | | |

Beispiel 3: Nichtionisches Tensid

In einem Kolben wurden 48 g D,L-Methionin, 348 g Wasser und 3,8 g wässriger $NH_3$-Lösung (25%ig) sowie 0,32 g Tween 65 vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 151 g D,L-Methionin, 1087 g Wasser, 11,8 g wässriger $NH_3$-Lösung (25%ig) sowie 1,0 g Tween 65 gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das D,L-Methionin abfiltriert und mit 300 ml Aceton gewaschen. Nach Trocknung des D,L-Methionins wurde das Schüttgewicht bestimmt.

Es traten 0 ml Schaum auf und das Schüttgewicht des isolierten D,L-Methionins lag bei 578 g/l.

Beispiel 4: Konzentrationsabhängige Experimente

In einem Kolben wurden 48 g D,L-Methionin, 348 g Wasser und 3,8 g wässrige $NH_3$-Lösung (25%ig) vorgelegt und bei einer Temperatur von 40°C im Kreislauf über einen Wärmetauscher umgepumpt. Zu dieser Suspension wurde mit einer Geschwindigkeit von 18 ml/min eine auf 90 °C erhitzte Lösung aus 151 g D,L-Methionin, 1087 g Wasser und 11,8 g wässrige $NH_3$-Lösung (25%ig) gegeben, wobei die Temperatur der vorgelegten Suspension bei 40°C gehalten wurde. Nach Zugabe von 650 ml der heißen Lösung wurden 500 ml Suspension entnommen und anschließend weitere 500 ml der heißen Lösung mit einer Geschwindigkeit von 18 ml/min zudosiert. Die entstandene Suspension wurde abgelassen, die Schaummenge bestimmt, das D,L-Methionin abfiltriert und mit 300 ml Aceton gewaschen. Nach Trocknung des D,L-Methionins wurde das Schüttgewicht bestimmt.

Die Kristallisationsversuche wurden in Gegenwart der folgenden Zusätze 8, 9 bzw. 10 (gem. Beispiel 1) durchgeführt, wobei die angegebene Konzentration durch Zugabe des Zusatzes in beiden Startlösungen/-suspensionen gemäß Tabelle 4 eingestellt wurde.

Zusatz 8) (7) + (2)

Zusatz 9) (7) + (3)

Zusatz 10) (7) + (4)

Die Konzentrationsangabe in Tabelle 3 gibt den Gesamtwirkstoffgehalt des Zusatzes ohne Wasser bezogen auf die Gesamtmasse der Lösung bzw. Suspension an.

Tabelle 3:

| Nr. | Zusatz | Zusatz-Konzentration (ppm) 1) | Schaummenge (ml) | D,L-Methionin-Schüttgewicht (g/l) |
|---|---|---|---|---|
| 1 | 8 | 100 | 350 | 522 |
| 2 | 8 | 200 | 180 | 561 |
| 3 | 8 | 400 | 5 | 610 |
| 4 | 8 | 800 | 0 | 630 |
| 5 | 8 | 1200 | 0 | 624 |
| 6 | 8 | 2000 | 0 | 615 |
| 7 | 8 | 4000 | 0 | 616 |
| 8 | 9 | 100 | 450-500 | 529 |
| 9 | 9 | 200 | 200 | 585 |
| 10 | 9 | 400 | 20-30 | 625 |
| 11 | 9 | 800 | 0 | 634 |
| 12 | 9 | 1200 | 0 | 657 |
| 13 | 9 | 2000 | 0 | 630 |
| 14 | 9 | 4000 | 0 | 601 |
| 15 | 10 | 100 | 350 | 507 |
| 16 | 10 | 200 | 150 | 558 |
| 17 | 10 | 400 | 80 | 614 |
| 18 | 10 | 800 | 20-30 | 630 |
| 19 | 10 | 1200 | 20-30 | 652 |
| 20 | 10 | 2000 | 5-10 | 650 |
| 21 | 10 | 4000 | 0 | 636 |
| 1)in der Lösung und/oder Suspension (analog Beispiel 1) | | | | |

Man erkennt, dass die erfindungsgemäßen Zusätze (mit Entschäumer) über den Konzentrationsbereich von 400 bis 4000 ppm das Schüttgewicht des D,L-Methionins auf Werte > 600 g/l verbessern.

## Patentansprüche

1. Verfahren zur Kristallisation von D,L-Methionin aus D,L-Methionin und D,L-Methioninammoniumsalz enthaltenden wässrigen Lösungen und/oder Suspensionen mit einem Met-Gehalt von 70 - 180 g/kg der Lösung und/oder Suspension, vorzugweise 90 - 150 g/kg der Lösung und/oder Suspension, einem $NH_4^+$-Gehalt von 1 - 5 g/kg der Lösung und/oder Suspension, in Gegenwart eines Kristallisationsadditivs,
welches ein nichtionisches oder anionisches Tensid oder eine Mischung aus verschiedenen nichtionischen oder anionischen Tensiden umfasst,
bei dem die Temperatur der Lösung und/oder Suspension von $T_1$ = 85-110°C direkt oder schrittweise auf $T_2$ = 30

- 50°C abgesenkt wird, so dass D,L-Methionin als Feststoff ausfällt.

**2.** Verfahren nach Anspruch 1, **dadurch gekennzeichnet, dass** das zur Bildung des Kristallisationsadditiv eingesetzte anionische Tensid eine der in Formel 1 bis 3 dargestellten Verbindungen, oder eine Mischung daraus ist:

$R^1$-O-$SO_3M$ (Formel 1)

$R^2$-O-$(CH_2)_n$-$SO_3M$ (Formel 2)

$R^3$-$(OC_2H_4)_n$-O-$SO_3M$ (Formel 3)

wobei n eine ganze Zahl von 1 bis 10, M Natrium oder Kalium und $R^1$, $R^2$ und $R^3$ eine lineare, verzweigte oder zyklische, gesättigte oder ungesättigte $C_8$-bis $C_{20}$-Alkylgruppe oder eine Arylgruppe ist.

**3.** Verfahren nach Anspruch 2, **dadurch gekennzeichnet, dass** das n gleich 2 und $R^1$, $R^2$ und $R^3$ lineare, gesättigte $C_8$-bis $C_{18}$-Alkylgruppen sind.

**4.** Verfahren gemäß Anspruch 2 oder 3, **dadurch gekennzeichnet, dass**
$C_nH_{2n+1}$-O-$SO_3Na$, mit n = 12 bis 18,
$C_nH_{2n+1}$-O-$C_2H_4$-$SO_3Na$, mit n = 8 bis 18 oder
$C_nH_{2n+1}$-$(OC_2H_4)_2$-O-$SO_3Na$, mit n = 12
als anionisches Tensid eingesetzt werden.

**5.** Verfahren nach Anspruch 1, **dadurch gekennzeichnet, dass** das nichtionische Kristallisationsadditiv ein Sorbitanfettsäureester oder eine Mischung aus verschiedenen Sorbitanfettsäureestern ist.

**6.** Verfahren nach Anspruch 4, **dadurch gekennzeichnet, dass** der Sorbitanfettsäureester gemäß Formel 4

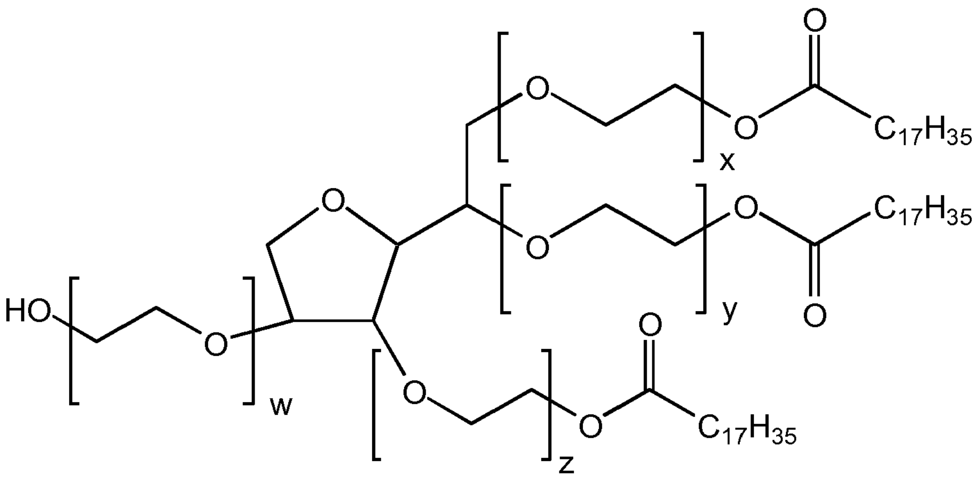

,

wobei w+x+y+z=20,
verwendet wird.

**7.** Verfahren nach einem der Ansprüche 1 bis 6, **dadurch gekennzeichnet, dass** die Konzentration des Kristallisationsadditivs in der Lösung und/oder Suspension, aus der die Kristallisation erfolgt, mindestens 700 ppm und maximal 4000 ppm, bezogen auf die Gesamtmasse der Lösung und/oder Suspension beträgt, vorzugsweise mindestens 750 ppm und maximal 2000 ppm, besonders bevorzugt mindestens 800 ppm und maximal 1000 ppm.

**8.** Verfahren nach einem der Ansprüche 1 bis 5, **dadurch gekennzeichnet, dass** die Lösung, aus der die Kristallisation erfolgt, zusätzlich einen Entschäumer enthält.

**9.** Verfahren nach Anspruch 6, **dadurch gekennzeichnet, dass** der Entschäumer Silikonöl enthält.

**10.** Verfahren gemäß Anspruch 8 oder 9, **dadurch gekennzeichnet, dass** das Gewichtsverhältnis Entschäumer: Kristallisationsadditiv auf Basis des Wirkstoffs im Bereich von 4:1 bis 1:1, vorzugsweise im Bereich von 3:1 bis 2:1 liegt und dabei die Konzentration des Kristallisationsadditivs mindestens 50 ppm und maximal 1200 ppm, bezogen auf die Gesamtmasse der Lösung und/oder Suspension beträgt, vorzugsweise 100 ppm bis 600 ppm, besonders bevorzugt 200 ppm bis 400 ppm.

**11.** Verfahren nach einem der Ansprüche 1 bis 10, **dadurch gekennzeichnet, dass** die Kristallisation durch Einleiten einer 85 bis 110°C heißen D,L-Methionin und D,L-Methioninammoniumsalz enthaltenden wässrigen Lösung und/oder Suspension in eine 30 bis 50°C warme D,L-Methionin und D,L-Methioninammoniumsalz enthaltende wässrige Lösung und/oder Suspension erfolgt, wobei die Temperatur der dabei entstehenden Mischung konstant zwischen 30 und 50°C gehalten wird.

**12.** Verfahren gemäß Anspruch 11, **dadurch gekennzeichnet, dass** die Kristallisation durch Einleiten einer 85 bis 110°C heißen D,L-Methionin und D,L-Methioninammoniumsalz enthaltenden Lösung in eine 30 bis 50°C warme D,L-Methionin und D,L-Methioninammoniumsalz enthaltende Suspension erfolgt.

**13.** Verfahren nach einem der Ansprüche 1 bis 10, **dadurch gekennzeichnet, dass** die Kristallisation zweistufig durchgeführt wird, wobei in der ersten Kristallisationsstufe eine 85 bis 110°C heiße D,L-Methionin und D,L-Methioninammoniumsalz enthaltende Lösung und/oder Suspension in eine 60 bis 80 °C warme D,L-Methionin und DL-Methioninammoniumsalz enthaltende Suspension eingeleitet und die Temperatur der dabei entstehenden Mischung konstant zwischen 60 und 80 °C gehalten wird und wobei die in der ersten Kristallisationsstufe erhaltene 60 bis 80 °C warme D,L-Methionin und D,L-Methioninammoniumsalz enthaltende Suspension in einer zweiten Kristallisationsstufe in eine 30 bis 50 °C warme D,L-Methionin und D,L-Methioninammoniumsalz enthaltende Suspension eingeleitet wird, wobei die Temperatur der dabei entstehenden Mischung konstant zwischen 30 und 50 °C gehalten wird.

**14.** Verfahren nach einem der Ansprüche 1 bis 13, **dadurch gekennzeichnet, dass** die Kristallisation durch Vakuumkristallisation erfolgt, wobei der Druck in der Kristallisationsstufe 60 bis 1000 mbar und, falls eine zweistufige Kristallisation durchgeführt wird, in der zweiten Kristallisationsstufe 35 bis 200 mbar beträgt.

**15.** Verfahren nach einem der Ansprüche 1 bis 14, **dadurch gekennzeichnet, dass** die D,L-Methionin- und D,L-Methioninammoniumsalze enthaltenden wässrigen Lösungen und/oder Suspensionen zuvor durch Auflösen und/oder Suspendieren von D,L-Methionin in Wasser in Gegenwart von entsprechenden Mengen Ammoniak hergestellt wurden.

**16.** Verfahren nach Anspruch 15, **dadurch gekennzeichnet, dass** zum Auflösen D,L-Methionin mit einem Methioningehalt von mindestens 90 Gew.-%, vorzugsweise mindestens 95 Gew.-%, verwendet wird.

**17.** Verfahren gemäß Anspruch 16, **dadurch gekennzeichnet, dass** D,L-Methionin in Form von Reinmethionin und/oder Rohmethionin aus einem beliebigen Herstellungsprozess mit einer Restfeuchte von 0,1 bis 9,5 Gew.-%, vorzugsweise 0,2 bis 4,5 Gew.-% verwendet wird.

**18.** Verfahren zur Herstellung von D,L-Methionin, bei dem zunächst durch Hydrolyse von D,L-Methioninnitril und/oder D,L-Methioninamid eine wässrige Lösung von D,L-Methioninammoniumsalz gebildet wird und daraus durch teilweise Entfernung des Ammoniaks aus dem D,L-Methioninammoniumsalz eine D,L-Methionin und D,L-Methioninammoniumsalz enthaltende wässrige Lösung und/oder Suspension erzeugt wird, aus der dann Methionin durch Kristallisation gemäß einem der Ansprüche 1 bis 17 gewonnen wird.

**19.** Verfahren gemäß Anspruch 18, **dadurch gekennzeichnet, dass** die wässrige Lösung von D,L-Methioninammoniumsalz vor der Kristallisation durch Eindampfen und/oder durch Abstrippen von Ammoniak auf einen $NH_4^+$-Gehalt von 1 - 5 g/kg der Lösung und/oder Suspension, vorzugsweise von 1,5 bis 3,0 g/kg, gebracht wird.

**20.** Verfahren gemäß Anspruch 18 oder 19, **dadurch gekennzeichnet, dass** die wässrige Lösung und/oder Suspension von D,L-Methionin und D,L-Methioninammoniumsalz durch Zugabe von Wasser und/oder D,L-Methionin auf einen Met-Gehalt von 70 - 180 g/kg der Lösung und/oder Suspension, vorzugsweise von 90 bis 150 g/kg, gebracht wird.

**21.** Verfahren nach einem der Ansprüche 1 bis 20, **dadurch gekennzeichnet, dass** das ausgefallene D,L-Methionin von der Mutterlauge getrennt und getrocknet oder zunächst umkristallisiert und nach Abtrennung von der dabei entstehenden Mutterlauge getrocknet wird.

**22.** Verfahren nach Anspruch 21, **dadurch gekennzeichnet, dass** die Mutterlauge in eine Kristallisationsstufe zurückgeführt wird.

**Figur 1**

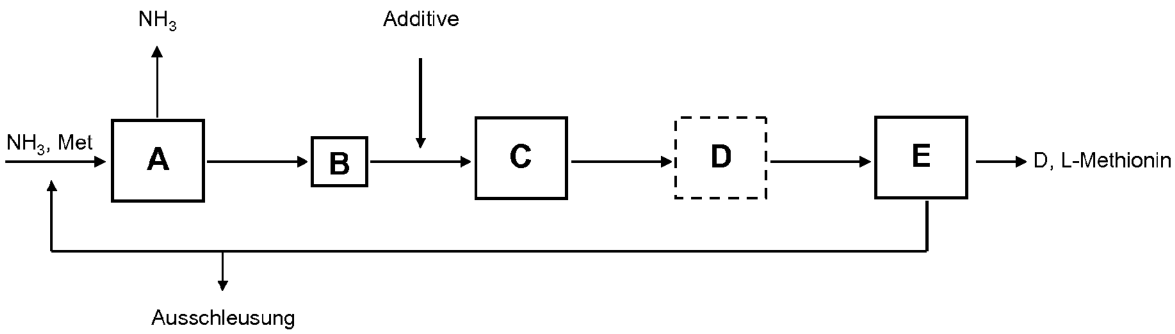

Europäisches Patentamt
European Patent Office
Office européen des brevets

# EUROPÄISCHER RECHERCHENBERICHT

Nummer der Anmeldung
EP 13 18 4831

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | WO 03/050071 A1 (DEGUSSA [DE]; WECKBECKER CHRISTOPH [DE]; KRULL HORST [DE]; BILZ JUERGE) 19. Juni 2003 (2003-06-19) | 1 | INV.<br>C07C319/20<br>C07C319/26<br>C07C319/28<br>C07C323/58 |
| A | * Ansprüche 1, 3, 8 * | 2-22 | |
| Y | "Dl-methionine crystallization", WPI / THOMSON,, Bd. 1971, Nr. 21, 1. Januar 1971 (1971-01-01), XP002681651, | 1,17 | |
| A | * Zusammenfassung * | 2-16, 18-22 | |
| | & JP 46- 019 610 B 1971 | | |
| Y | JP 11 158140 A (SUMITOMO) 15. Juni 1999 (1999-06-15) | 1,17 | |
| A | * Ansprüche 1, 3 * | 2-16, 18-22 | |
| Y,D | US 2005/176115 A1 (KOBAYASHI YOICHI [JP] ET AL) 11. August 2005 (2005-08-11) | 1,17 | |
| A | * Anspruch 1 *<br>* Seite 5; Beispiel 6 * | 2-16, 19-22 | **RECHERCHIERTE SACHGEBIETE (IPC)** |
| A | CANSELIER J P: "THE EFFECTS OF SURFACTANTS ON CRYSTALLIZATION PHENOMENA", JOURNAL OF DISPERSION SCIENCE AND TECHNOLOGY, TAYLOR AND FRANCIS GROUP, NEW YORK, NY, US, Bd. 14, Nr. 6, 1. Januar 1993 (1993-01-01), Seiten 625-644, XP008011857, ISSN: 0193-2691, DOI: 10.1080/01932699308943435<br>* Zusammenfassung * | 1-22 | C07C |

Der vorliegende Recherchenbericht wurde für alle Patentansprüche erstellt

1

| Recherchenort | Abschlußdatum der Recherche | Prüfer |
|---|---|---|
| Den Haag | 13. Februar 2014 | Guazzelli, Giuditta |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 13 18 4831

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-02-2014

| Im Recherchenbericht angeführtes Patentdokument | Datum der Veröffentlichung | Mitglied(er) der Patentfamilie | Datum der Veröffentlichung |
|---|---|---|---|
| WO 03050071 A1 | 19-06-2003 | AT 430725 T | 15-05-2009 |
| | | AU 2002321293 A1 | 23-06-2003 |
| | | BR 0214781 A | 09-11-2004 |
| | | CA 2469526 A1 | 19-06-2003 |
| | | CN 1599712 A | 23-03-2005 |
| | | DE 10160358 A1 | 18-06-2003 |
| | | EP 1451139 A1 | 01-09-2004 |
| | | ES 2325574 T3 | 09-09-2009 |
| | | JP 4213038 B2 | 21-01-2009 |
| | | JP 2005511733 A | 28-04-2005 |
| | | KR 20040061024 A | 06-07-2004 |
| | | MX PA04005443 A | 24-02-2006 |
| | | RU 2294922 C2 | 10-03-2007 |
| | | US 2005131111 A1 | 16-06-2005 |
| | | WO 03050071 A1 | 19-06-2003 |
| | | ZA 200404479 A | 22-02-2006 |
| JP 11158140 A | 15-06-1999 | KEINE | |
| US 2005176115 A1 | 11-08-2005 | AU 2003281546 A1 | 09-02-2004 |
| | | CN 1671855 A | 21-09-2005 |
| | | EP 1541691 A1 | 15-06-2005 |
| | | US 2005176115 A1 | 11-08-2005 |
| | | WO 2004009829 A1 | 29-01-2004 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20050176115 A
- JP 2004254690 A
- DE 60127538
- WO 2008006977 A
- WO 2007034065 A
- DE 10238212
- WO 2003050071 A